# EUROPEAN PATENT APPLICATION

(11) **EP 2 620 173 A1**
(43) Date of publication of application: **31.07.2013**
(21) Application number: 12153045.5
(22) Date of filing: 30.01.2012
(51) Int. Cl.: A61M 5/168, A61M 5/36

(54) **Intravenous infusion pump system**

(71) Applicant: Heartware BVBA, 3140 Keerbergen (BE)
(72) Inventor: Janssenswillen, Eddy, 3140 Keerbergen (BE); Janssenswillen, Timothy, 3140 Keerbergen (BE)
(74) Representative: Plas, Axel Ivo Michel

(57) **Abstract**

The present invention relates to an intravenous infusion pump system (100), comprising a pump (101) which is designed to pump the fluid or blood through the fluid or blood conduit (110a, 110b) at a flow rate of at least 1 litre per hour, and comprising a processor (102) which is adapted to control said pump (101) to operate in three possible configurations, wherein either the fluid or blood is administered to the patient at a maximum flow rate of the pump (101), either a bolus volume (BOL VOL) of fluid or blood has to be administered to the patient in a time interval (TIME), both the bolus volume (BOL VOL) and the time interval (TIME) being set by the operator via said user interface (103); either a bolus volume (BOL VOL) of fluid or blood is administered to the patient with respect for a maximum infusion pressure (PMAX), both the bolus volume (BOL VOL) and the maximum infusion pressure (PMAX) being set by the operator via said user interface (103), wherein the flow rate of the pump (101) is raised until the set maximum infusion pressure (PMAX) is reached, whereafter the flow rate of the pump (101) is fine-tuned under the control of said processor (102) in function of the pressure sensed by said pressure sensor (104).

## Description

### Field of the Invention

The present invention generally relates to an intravenous infusion pump system.

More particularly, the present invention relates to an intravenous infusion pump system, comprising
- a pump with a controllable flow rate which is connected to a container holding fluid or blood to be infused in a patient through a fluid or blood conduit connected to the patient by means of an intravenous access device;
- a processor adapted to control said pump;
- a user interface enabling an operator of the intravenous infusion system to set parameters enabling said processor to control said pump;
- at least one pressure sensor positioned between said pump and said intravenous access device, and adapted to sense pressure of the fluid or blood present in the fluid or blood conduit between said pump and said intravenous access device;
- at least one air sensor positioned between said container and said pump, and adapted to detect air in the fluid or blood present in the fluid or blood conduit between said container and said pump.

### Background of the Invention

Intravenous therapy or IV therapy is the giving of substances directly into a vein. Intravenous therapy can range from an intravenous infusion with or without an infusion pump, intravenous cannula with an injection port, through to a central venous line. This therapy provides a direct route to the bloodstream which allows for hydration, administration of blood or blood products and administration of medications.

A wide variety of intravenous infusion systems have been developed over the years that are capable of administering medication and/or other fluids intravenously into the body.

An existing solution consists of a drip chamber which is able to estimate the flow rate and prevents air bubbles from entering the blood by placing the fluid container above the level of the patient. To regulate the drip rate, a clamp is used.

This system however is disadvantageous since there is a poor control of the flow rate and the volume. It requires regular verification by a doctor or a nurse. Since the need of regular verification, this system often leads to unintentional overfilling of the patient. Furthermore, there is a poor prevention of air embolism and poor pressure control.

In the operation room, the intensive care unit or an emergency unit, it is often necessary that the intravenous infusion system allows fast fluid restitution, wherein a considerable amount of fluid has to be administered in a short period of time. With heavily bleeding patients for instance, the system has to be able to administer a large amount of blood in a very short time period intravenously to the patient in order to avoid that the patient bleeds to death.

At present, when there is an emergency situation, for instance a patient which is bleeding to death, several perfusionists manually give pressure to a blood container in order to administer the necessary amount of blood as quick as possible to the patient.

A further known system that can be used if the patient requires a high flow rate and the intravenous access device is of a large enough diameter to accommodate it, is a rapid infuser. This is an inflatable cuff or a similar electrical device which is placed around the fluid or blood container and which is connected to a compressed air source to force the fluid or blood into the patient.

Such a rapid infuser however has a limited control of the flow rate and the volume. It is inconvenient to place, and since it needs regular verification, it often leads to unintentional overfilling of the patient. It has also the disadvantage that there is a poor prevention of air embolism and there is a poor pressure control.

At present, already different kinds of intravenous infusion pump systems are known, such a system being provided with a pump that controls the flow rate and total amount of fluid delivered to a patient.

A first problem with the known intravenous infusion pump systems is the prevention of air bubbles in the fluid to be administered. Since it could be life-threatening to have air bubbles being delivered with the administered fluid, in this way leading to air entering into the blood stream of the body, also called air embolism, which can have potentially harmful consequences and in the worst case (3 - 8 millilitres per kilogram of body weight) can lead to a stroke or even death, intravenous infusion pump systems have now widely employed gas bubble detectors that detect gas bubbles in a fluid prior to its delivery in the blood stream.

Another problem occurring with intravenous infusion pump systems is the ability to control the pressure in the line between the infusion pump and the intravenous access device serving to administer the fluid or blood into the body of the patient. For this purpose, it is already known to use pressure sensors. The intravenous infusion pump system furthermore has to be able to sense a fluid or blood blockage. Therefore, the pressure sensor has to be able to detect the increase of the pressure in the tubing that occurs as a result of an occlusion or blockage downstream of the pump.

Still another problem of intravenous infusion pump systems is the ability to achieve flow rates which are high enough to administer a bolus volume of fluid or blood in emergency situations. A bolus volume is a volume of fluid or blood which is administered to raise its concentration in the blood of a patient to an effective level.

Exemplary intravenous infusion pump systems are amongst others described in US 6, 475, 178 B1 and BR PI0904983 A2.

All of the known prior art intravenous infusion pump systems however suffer from the drawback that these do not allow a pressure controlled large fluid restitution, and thus are not able to administer safely a large amount of fluid, blood included, in a short time interval at flow rates of more than 1 litre per hour. It is therefore an object of the present invention to provide an intravenous infusion pump system which allows a pressure controlled large fluid restitution.

### Summary of the Invention

The object of the invention is solved by providing an intravenous infusion pump system, comprising
- a pump with a controllable flow rate which is connected to a container holding fluid or blood to be infused in a patient through a fluid or blood conduit connected to the patient by means of an intravenous access device;
- a processor adapted to control said pump;
- a user interface enabling an operator of the intravenous infusion system to set parameters enabling said processor to control said pump;
- at least one pressure sensor positioned between said pump and said intravenous access device, and adapted to sense pressure of the fluid or blood present in the fluid or blood conduit between said pump and said intravenous access device;
- at least one air sensor positioned between said container and said pump, and adapted to detect air in the fluid or blood present in the fluid or blood conduit between said container and said pump;
wherein said pump is designed to pump the fluid or blood through the fluid or blood conduit at a flow rate of at least 1 litre per hour;
and in that the processor is adapted to control said pump to operate in the following possible configurations:
- a first configuration wherein the fluid or blood is administered to the patient at a maximum flow rate of the pump;
- a second configuration wherein a bolus volume of fluid or blood (BOL VOL) has to be administered to the patient in a time interval (TIME), both the bolus volume (BOL VOL) and the time interval (TIME) being set by the operator via said user interface; and
- a third configuration wherein a bolus volume (BOL VOL) of fluid or blood is administered to the patient with respect for a maximum infusion pressure (PMAX), both the bolus volume (BOL VOL) and the maximum infusion pressure (PMAX) being set by the operator via said user interface, wherein the flow rate of the pump is raised until the set maximum infusion pressure (PMAX) is reached, whereafter the flow rate of the pump is fine-tuned under the control of said processor in function of the pressure sensed by said pressure sensor;
and in that said processor is adapted to stop said pump automatically in each case when:
- said pressure sensor senses a pressure in the fluid or blood conduit between said pump and said intravenous access device exceeding a pre-programmed absolute maximum pressure of the intravenous infusion pump system; and/or
- said air sensor senses the presence of air in the fluid or blood conduit between said container and said pump.

The most important advantage of the intravenous infusion pump system according to the invention in view of known intravenous infusion systems is that this system, in addition to the prevention of accidental air infusion leading to air embolism and the prevention of overpressures, allows a pressure controlled large fluid restitution. In this way, the system according to the invention provides the possibility to rapidly restore the patient's circulating fluid and blood volume, which is of major importance in case of important blood loss.

A further advantage of the intravenous infusion pump system in view of known intravenous infusion systems is that there is a better controlled fluid or blood management, through which inadvertent overfilling often seen with existing intravenous infusion systems requiring permanent attention is avoided.

Furthermore, with the intravenous infusion pump system according to the invention, the correct amount of fluid or blood can be safely delivered to the intended destination, and is thus precise and traceable.

In a preferred embodiment of an intravenous infusion pump system according to the invention, in the second and third configuration, said processor is adapted to set said pump to a basic flow rate (RATE), and is adapted to set said pump to return automatically to said basic flow rate (RATE) after the set bolus volume (BOL VOL) has been administered to the patient.

In an advantageous embodiment of an intravenous infusion pump system according to the invention, the pump is a peristaltic pump, also called roller pump. A peristaltic pump is a type of positive displacement pump used for pumping a variety of fluids. The fluid is contained within a flexible tube fitted inside a circular pump casing. A rotor with a number of rollers which are attached to the external circumference compresses the flexible tube. As the rotor turns, the part of tube under compression closes or occludes, in this way forcing the fluid to be pumped to move through the tube. Additionally, as the tube opens to its natural state after the passing of the cam, called restitution or resilience, the fluid flow is induced to the pump.

The pressure sensor preferably is a non-invasive pressure sensor, more preferably a non-invasive pressure-occlusion detector.

The air sensor preferably is a non-invasive air sensor, more preferably a non-invasive drip chamber level sensor.

Non-invasive sensors are sensors which do not enter the sterile zone. Non-invasive sensors are preferred above invasive detectors since non-invasive sensors allow easy mounting of the sensors around the conduit of the fluid or blood. Furthermore, less time is needed to prime the intravenous infusion pump system.

The non-invasive pressure-occlusion detector accurately monitors tubing occlusion. The advantage of such a detector is that it is a free-entry, maintenance-free sensor and it detects positive and negative air pressure in soft tubing and produces a corresponding output signal.

A non-invasive drip chamber level sensor is a sensor which detects air bubbles, using for instance pulse-type ultrasonic technology or light intensity. The advantage of using ultrasonic air bubble detection sensors is that they accurately and repeatedly detect unwanted air even within blood, which can coat the inside of tubing lines, potentially masking air bubbles from other types of sensing technologies, and thus provide highly reliable, non-invasive air bubble detection capabilities in the most critical applications where the safety of a patient is mandatory. Non-invasive drip chamber level sensors are usable with soft, squeezable drip chambers and tubing in a wide range of diameters and tapers. Advantages of non-invasive drip chamber level sensors is that they resist the effects of drip chamber relaxation, slippage and vibration, which would normally cause false air alarms.

In a favourable embodiment of an intravenous infusion system according to the invention, said user interface comprises a monitor which is adapted to permanently display an actual volume of infused fluid or blood (ACT VOL).

Said monitor preferably is a touch screen based monitor.

In a preferred embodiment of an intravenous infusion pump system according to the invention, said pre-programmed absolute maximum pressure is adjustable by the installer of the intravenous infusion pump system.

Said pre-programmed absolute maximum pressure preferably is approximately 500 mm Hg.

The invention furthermore relates to disposable tubing for use in a conduit of an intravenous infusion pump system according to the present invention as described above.

The invention also relates to a drip chamber level sensor for use in an intravenous infusion pump system according to the present invention as described above.

### Brief Description of the Drawings

Fig. 1 illustrates a schematic representation of an intravenous infusion pump system according to the invention;

Fig. 2 illustrates a drip chamber level sensor according to the invention; and

Fig. 3 illustrates a pressure-occlusion sensor according to the invention.

### Detailed Description of Embodiment(s)

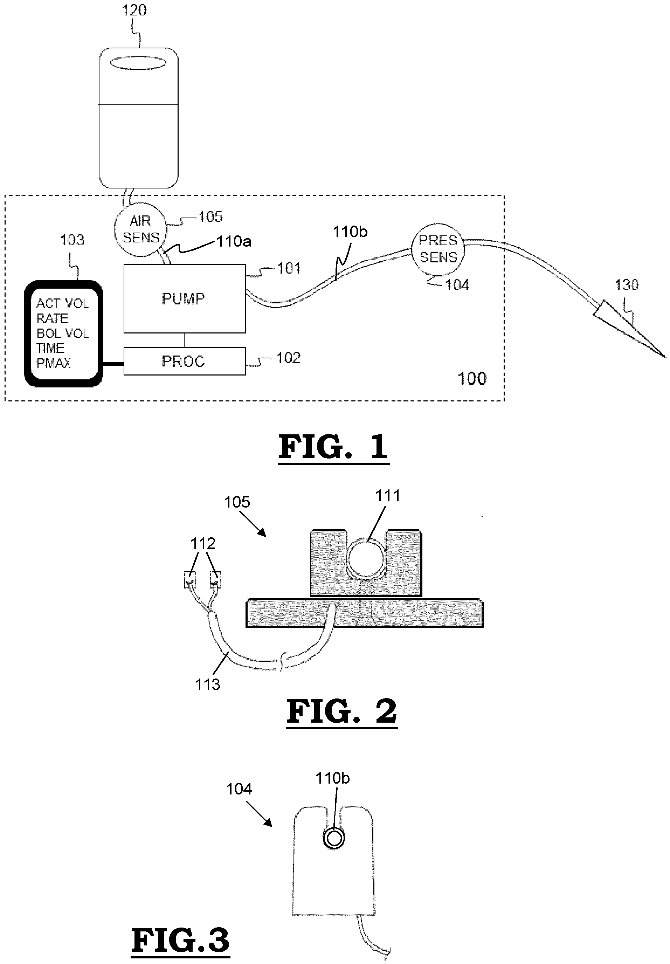

Figure 1 shows a preferred embodiment of an intravenous infusion pump system (100) according to the present invention for infusing fluid or blood to the body of a patient.

The intravenous infusion pump system (100) comprises a pump (101) with a controllable flow. The pump (101) preferably is a peristaltic or roller pump.

This pump (101) is connected to a container (120) holding a fluid or blood which has to be infused to a patient by means of an intravenous access device (130). The container (120) preferably is a pre-filled, batch container consisting of sterile glass bottle, plastic bottle or plastic bag. The container (120) thus has a limited capacity and is configured for discrete and not continuous functioning, i.e. it is filled before use with a determined quantity of infusion fluid or blood, it is emptied during use while supplying the infusion fluid or blood to the patient and it is replaceable time-by-time as the container (120) is emptied.

The pump (101) is designed to pump the fluid or blood through the fluid or blood conduit (110a, 110b) at a flow rate of at least 1 litre per hour.

The simplest form of the intravenous access device (130) is a hypodermic needle which is a hollow needle which passes through the skin directly into the vein of the body of a patient. This needle then can directly be connected to a syringe to deliver fluid or blood into the bloodstream of the patient, or can be connected to a length of tubing and thence whichever collection or infusion system is desired. The most convenient site is often the arm, especially the veins on the back of the hand, or the median cubital vein at the elbow, but any identifiable vein can be used.

The most common intravenous access device (130) which is used in both hospitals and pre-hospital services is a peripheral intravenous line (PVC or PIV) consisting of a short catheter of a few centimeters long which is inserted through the skin into a peripheral vein being any vein not inside the chest or abdomen. This is usually in the form of a cannula-over-needle device, in which a flexible plastic cannula comes mounted on a metal trocar. Once the tip of the needle and the cannula are located in the vein, the trocar is withdrawn and discarded and the cannula advanced inside the vein to the appropriate position and secured. Any accessible vein can be used although, for adults, the arm and hand veins are used most commonly and for infants, the scalp veins are sometimes used.

Other possible intravenous access devices (130) are:
- central intravenous lines which flow through a catheter with its tip within a large vein, usually being the superior vena cava or inferior vena cava or within the right atrium of the heart;
- peripherally inserted central catheter (PICC) being a form of an intravenous access device (130) that can be used for a prolonged period of time, e.g. for long chemotherapy regimens, extended antibiotic therapy or total parental nutrition especially in chronically ill patients, wherein the PICC line is inserted through a sheath into a peripheral vein, usually in the arm, and then carefully advanced upward until the catheter is in the superior vena cava or the right atrium of the heart;
- central venous catheters, also called central line, CVC, central venous line or central venous access catheter, being a catheter which is placed into a large vein in the neck, chest or groin, and consisting of different types including
   - non-tunneled catheters which are fixed in place at the site of insertion, with the catheter and attachments protruding directly;
   - tunneled catheters which are passed under the skin from the insertion site to a separate exit site, where the catheter and its attachments emerge from underneath the skin;
   - an implanted port which is similar to a tunneled catheter but which is left entirely under the skin.

The container (120), the pump (101) and the intravenous access device (130) are connected to each other by means of a conduit (110a, 110b) which at a first end is connected to the container (120) and at a second end is connected to the intravenous access device (130) in order to supply the infusion fluid or blood to the patient. This conduit (110a, 110b) is substantially made out of tubing. This tubing preferably is disposable tubing which is made out of silicon.

Between the container (120) and the pump (101), one or more non-invasive air sensors (105), preferably in the form of a drip chamber level sensor, are provided. Such a non-invasive drip chamber level sensor (105) is shown in figure 2. As can be seen in figure 2, this non-invasive air sensor (105) is mounted around a drip chamber (111), which also forms part of the conduit (110a) between the container (120) and the pump (101). This air sensor (105) is adapted to detect air bubbles in the infused fluid or blood present in this drip chamber (111). The drip chamber level sensor (105) is connected to the drip chamber (111) by means of two connectors (112) via a dual coaxial cable (113). In the embodiment as shown in figure 1, one such air sensor (105) is provided.

Furthermore, between the pump (101) and the intravenous access device (130), one or more non-invasive pressure sensors (104), in the form of a pressure-occlusion detector, are provided. Such a non-invasive pressure-occlusion sensor (104) is shown in figure 3. As can be seen in figure 3, this non-invasive pressure sensor (104) is placed around the fluid or blood conduit (110b) which is situated between the pump (101) and the intravenous access device (130) and is adapted to sense pressure and an occlusion of the infused fluid or blood present in this conduit (110b). The pressure sensor (104) is connected to the processor (102) via a shielded cable. In the embodiment as shown in figure 1, one such non-invasive pressure-occlusion sensor (104) is provided.

This pressure sensor (104) also senses the temperature of the fluid or blood present in the respective fluid or blood conduit (110b), since the hardness of the tubing of the conduits (110a, 110b) depends on the temperature. An algorithm is used to correct for these changes. In this way, the accuracy of the intravenous infusion pump system (100) is increased.

Examples of the pressure sensor (104) and the air sensor (105) are the non-invasive drip chamber level sensor and the pressure-occlusion sensor of the firm Introtek®.

The intravenous infusion pump system (100) according to the invention furthermore is provided with a user interface (103) enabling the operator of the intravenous infusion pump system (100) to set parameters enabling a processor (102) to control the pump (101). This user interface (103) preferably is a touch screen based monitor. This touch screen based monitor (103) preferably is built in into the pump (101), and the processor (102) preferably is a microprocessor which is applied onto a print plate incorporated in the pump (101).

On this touch screen based monitor (103), the operator can give in the following parameters in order to enable the processor (102) to control the pump (101):
- a basic flow rate (RATE) of the pump (101);
- a bolus volume (BOL VOL) of fluid or blood that has to be administered to the patient;
- a time interval (TIME) wherein a bolus volume (BOL VOL) has to be administered to the patient;
- a maximum infusion pressure (PMAX).

Furthermore, the touch screen based monitor (103) permanently displays the actual volume (ACT VOL) of infused fluid or blood.

The processor (102) receives signals from the air sensor (105) and the pressure sensor (104). Depending on the protocol installed between these sensors (104, 105) and the processor (102), the processor (102) receives signals from the sensors (104, 105) continuously, in certain time intervals or when the processor (102) triggers the sensor(s) (104, 105) to receive signals.

The processor (102) furthermore is adapted to control said pump (101) to operate in three different possible configurations.

In a first configuration, the fluid or blood is administered to the patient at a maximum flow rate of the pump (101). In this case, the pump (101) will administer fluid or blood to the patient as long as there is fluid or blood present in the container (120). If the container (120) is empty, and the air sensor (105) consequently senses air in the drip chamber, the processor (102) will stop the pump (101) automatically. Preferably, at that moment an alarm will be sounded.

In a second configuration, a bolus volume (BOL VOL) of fluid or blood has to be administered to the patient in a time interval (TIME), both the bolus volume (BOL VOL) and the time interval (TIME) being set by the operator via said user interface (103). After the set bolus volume (BOL VOL) has been administered to the patient, said processor (102) is adapted to control said pump (101) to automatically return to the basic flow rate (RATE).

In a third configuration, a bolus volume (BOL VOL) of fluid or blood is administered to the patient with respect for a maximum infusion pressure (PMAX), both the bolus volume (BOL VOL) and the maximum infusion pressure (PMAX) being set by the operator via said user interface (103). The flow rate of the pump (101) is therewith raised until the set maximum infusion pressure (PMAX) is reached, whereafter the flow rate of the pump (101) is fine-tuned under the control of said processor (102) in function of the pressure sensed by said pressure sensor (104). A maximum margin of error of the pressure of 5% on 100 mm Hg is therewith allowed. Also in this case, after the set bolus volume (BOL VOL) has been administered to the patient, said processor (102) is adapted to control said pump (101) to automatically return to the basic flow rate (RATE).

In each case, if the processor (102) receives a signal of the pressure sensor (104) that the pressure in the fluid or blood conduit (110b) between the pump (101) and the intravenous access device (130) exceeds a pre-programmed absolute maximum pressure of the intravenous infusion pump system (100), the processor (102) is adapted to stop said pump (101) automatically. Also in the case that the air sensor (105) senses the presence of air in the fluid or blood conduit (110a) between the container (120) and the pump (101), the processor (102) automatically stops said pump (101). Preferably, an alarm will be produced by the processor (102) or the sounding of an alarm is controlled by the processor (102) when the pump (101) is stopped automatically. When the pump (101) is stopped, the fluid or blood conduit (110a, 110b) is clamped such that no fluid can be moved anymore in this conduit (110a, 110b). When a roller pump is used, the clamping is performed by the roller(s) of the roller pump.

Said pre-programmed maximum pressure is adjustable by the installer of the intravenous infusion pump system (100) and is preferably 500 mm Hg.

Although the present invention has been illustrated by reference to the specific embodiment, it will be apparent to those skilled in the art that the invention is not limited to the details of the foregoing illustrative embodiments, and that the present invention may be embodied with various changes and modifications without departing from the scope thereof. The present embodiments are therefore to be considered in all respects as illustrative and not restrictive, the scope of the invention being indicated by the appended claims rather than by the foregoing description, and all changes which come within the meaning and range of equivalency of the claims are therefore intended to be embraced therein. In other words, it is contemplated to cover any and all modifications, variations or equivalents that fall within the scope of the basic underlying principles and whose essential attributes are claimed in this patent application. It will furthermore be understood by the reader of this patent application that the words "comprising" or "comprise" do not exclude other elements or steps, that the words "a" or "an" do not exclude a plurality, and that a single element, such as a computer system, a processor, or another integrated unit may fulfil the functions of several means recited in the claims. Any reference signs in the claims shall not be construed as limiting the respective claims concerned. The terms "first", "second", third", "a", "b", "c", and the like, when used in the description or in the claims are introduced to distinguish between similar elements or steps and are not necessarily describing a sequential or chronological order. Similarly, the terms "top", "bottom", "over", "under", and the like are introduced for descriptive purposes and not necessarily to denote relative positions. It is to be understood that the terms so used are interchangeable under appropriate circumstances and embodiments of the invention are capable of operating according to the present invention in other sequences, or in orientations different from the one(s) described or illustrated above

## Claims

1. An intravenous infusion pump system (100), comprising
- a pump (101) with a controllable flow rate which is connected to a container (120) holding fluid or blood to be infused in a patient through a fluid or blood conduit (110a, 110b) connected to the patient by means of an intravenous access device (130);
- a processor (102) adapted to control said pump (101);
- a user interface (103) enabling an operator of the intravenous infusion system (100) to set parameters enabling said processor (102) to control said pump (101);
- at least one pressure sensor (104) positioned between said pump (101) and said intravenous access device (130), and adapted to sense pressure of the fluid or blood present in the fluid or blood conduit (110b) between said pump (101) and said intravenous access device (130);
- at least one air sensor (105) positioned between said container (120) and said pump (101), and adapted to detect air in the fluid or blood present in the fluid or blood conduit (110a) between said container (120) and said pump (101);
**CHARACTERIZED IN THAT**
said pump (101) is designed to pump the fluid or blood through the fluid or blood conduit (110a, 110b) at a flow rate of at least 1 litre per hour;
and **in that** the processor (102) is adapted to control said pump (101) to operate in the following possible configurations:
- a first configuration wherein the fluid or blood is administered to the patient at a maximum flow rate of the pump (101);
- a second configuration wherein a bolus volume (BOL VOL) of fluid or blood has to be administered to the patient in a time interval (TIME), both the bolus volume (BOL VOL) and the time interval (TIME) being set by the operator via said user interface (103); and
- a third configuration wherein a bolus volume (BOL VOL) of fluid or blood is administered to the patient with respect for a maximum infusion pressure (PMAX), both the bolus volume (BOL VOL) and the maximum infusion pressure (PMAX) being set by the operator via said user interface (103), wherein the flow rate of the pump (101) is raised until the set maximum infusion pressure (PMAX) is reached, whereafter the flow rate of the pump (101) is fine-tuned under the control of said processor (102) in function of the pressure sensed by said pressure sensor (104);
and **in that** said processor (102) is adapted to stop said pump (101) automatically in each case when:
- said pressure sensor (104) senses a pressure in the fluid or blood conduit (110a) between said pump (101) and said intravenous access device (130) exceeding a pre-programmed absolute maximum pressure of the intravenous infusion pump system (100); and/or
- said air sensor (105) senses the presence of air in the fluid or blood conduit (110b) between said container (120) and said pump (101).

2. An intravenous infusion pump system (100) according to claim 1, **CHARACTERISED IN THAT** in the second and third configuration, said processor (102) is adapted to control said pump (101) to automatically return to a basic flow rate (RATE) after the set bolus volume (BOL VOL) has been administered to the patient.

3. An intravenous infusion pump system (100) according to claim 1 or 2, **CHARACTERISED IN THAT** said pump (101) is a peristaltic pump.

4. An intravenous infusion pump system (100) according to any one of claims 1 to 3, **CHARACTERISED IN THAT** the pressure sensor (104) is a non-invasive pressure sensor.

5. An intravenous infusion pump system (100) according to claim 4, **CHARACTERISED IN THAT** the pressure sensor (104) is a non-invasive pressure-occlusion detector.

6. An intravenous infusion pump system (100) according to any one of claims 1 to 5, **CHARACTERISED IN THAT** the air sensor (105) is a non-invasive air sensor.

7. An intravenous infusion pump system (100) according to claim 6, **CHARACTERISED IN THAT** the air sensor (105) is a non-invasive drip chamber level sensor.

8. An intravenous infusion pump system (100) according to any one of claim 1 to 7, **CHARACTERISED IN THAT** said user interface (103) is a monitor which is adapted to permanently display an actual volume (ACT VOL) of infused fluid or blood.

9. An intravenous infusion pump system (100) according to claim 7 or 8, **CHARACTERISED IN THAT** said monitor (103) is a touch screen based monitor.

10. An intravenous infusion pump system (100) according to any one of claims 1 to 9, **CHARACTERISED IN THAT** said pre-programmed absolute maximum pressure is adjustable by the installer of the intravenous infusion pump system (100).

11. An intravenous infusion pump system (100) according to any one of claims 1 to 10, **CHARACTERISED IN THAT** said pre-programmed absolute maximum pressure is approximately 500 mm Hg.

12. Disposable tubing for use in a conduit (110a, 110b) of an intravenous infusion pump system (100) according to any one of the preceding claims.

13. Drip chamber level sensor (105) for use in an intravenous infusion pump system (100) according to any one of the preceding claims.
